## Europäisches Patentamt

## European Patent Office

## Office européen des brevets

(19)

(11) Veröffentlichungsnummer: **0 078 993**
**B1**

(12) **EUROPÄISCHE PATENTSCHRIFT**

(45) Veröffentlichungstag der Patentschrift:
**29.01.86**

(21) Anmeldenummer: **82109963.7**

(22) Anmeldetag: **28.10.82**

(51) Int. Cl.⁴: **C 07 C  63/10,** C 07 C  51/60

(54) **Verfahren zur Herstellung von Benzoylchlorid.**

(30) Priorität: **07.11.81 DE 3144316**

(43) Veröffentlichungstag der Anmeldung:
**18.05.83 Patentblatt 83/20**

(45) Bekanntmachung des Hinweises auf die Patenterteilung:
**29.01.86 Patentblatt 86/5**

(84) Benannte Vertragsstaaten:
**DE FR GB IT NL**

(56) Entgegenhaltungen:
**EP - A - 0 019 341
DE - A - 1 070 616
DE - A - 1 909 523**

(73) Patentinhaber: **BAYER AG, Konzernverwaltung RP
Patentabteilung, D-5090 Leverkusen 1 Bayerwerk (DE)**

(72) Erfinder: **Neumann, Rainer, Dr.,
Bodelschwinghstrasse 16, D-4150 Krefeld (DE)**
Erfinder: **Morgenstern, Karl, Dr., Deswatinesstrasse 59,
D-4150 Krefeld (DE)**
Erfinder: **Lipper, Karl-August, Dr., Deswatinesstrasse 14,
D-4150 Krefeld (DE)**
Erfinder: **Brühne, Friedrich, Dr., Fabritiusstrasse 24,
D-4150 Krefeld (DE)**
Erfinder: **Böckmann, Walter, Dr., Fabritiusstrasse 19,
D-4150 Krefeld (DE)**
Erfinder: **Casper, Clemens, Dr., Giesenweg 76,
D-4150 Krefeld (DE)**
Erfinder: **Zingel, Siegfried, Dipl.-Ing., Birmesstrasse 46,
D-4150 Krefeld (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 078 993 B1

## Beschreibung

Die Erfindung betrifft ein Verfahren zur Herstellung von Benzoylchlorid.

Bei der Herstellung von Benzoesäure durch katalytische Oxidation von Toluol mit Sauerstoff oder Sauerstoff enthaltenden Gasen in flüssiger Phase (beschrieben z.B. in Ullmanns Enzyklopädie der technischen Chemie, 4. Auflage, Band 8, S. 367-369) fällt bei der Aufarbeitung des Oxidationsproduktes ein hochsiedender, teerartiger Rückstand an, der schwerlich einer weiteren Verwendung zugeführt werden kann. Dieser Rückstand, der bei der Aufarbeitung, d.h. in der Regel bei der destillativen Reinigung des Benzoesäure-haltigen Reaktionsproduktes, erhalten wird, besteht im wesentlichen aus restlicher Benzoesäure, Benzylbenzoat, verschiedenen isomeren Phenylbenzoesäuren, Diphenyl und verschiedenen Schwermetallsalzen, wie Kobalt- und Mangansalzen. Da dieser Rückstand in einer Menge von bis zu 10 %, bezogen auf die gewonnene, reine Benzoesäure anfällt, was bei den technisch üblichen Verfahren bis zu einigen 1000 t im Jahr ausmacht, ist es aus wirtschaftlichen Gründen lohnenswert, diesen Rückstand auf verwertbare Produkte hin aufzuarbeiten.

Es wurde nun ein Verfahren zur Herstellung von Benzoylchlorid gefunden, das dadurch gekennzeichnet ist, dass man den bei der katalytischen Oxidation von Toluol mit Sauerstoff oder Sauerstoff enthaltenden Gasen in flüssiger Phase erhaltenen, hochsiedenden Rückstand von vorhandenen Schwermetallsalzen weitgehend befreit, dann den Rückstand bei erhöhen Temperaturen, gegebenenfalls unter Einwirkung von Licht, chloriert und anschliessend das Benzoylchlorid abtrennt.

Nach dem erfindungsgemässen Verfahren wird der bei der katalytischen Oxidation von Toluol mit Sauerstoff oder Sauerstoff enthaltenden Gasen in üblicher Weise, wie z.B. durch Destillation des Oxidationsgemisches, erhaltene hochsiedende, teerartige Rückstand, der hauptsächlich aus Benzylbenzoat (ca. 70 bis 75 %) und Benzoesäure (ca. 10 bis 15 %), neben Phenylbenzoesäure, Diphenyl und verschiedenen Schwermetallsalzen, wie Kobalt- und Mangansalzen, die als solche der Oxidation als Katalysatoren zugesetzt werden, besteht, weitgehend von den Schwermetallsalzen befreit.

Beispielsweise kann man die Schwermetallsalze aus dem Rückstand durch Destillation bei Normal-, Unter- oder Überdruck abtrennen. Im allgemeinen wird die Destillation bei Temperaturen von etwa 120 bis 330 °C, vorzugsweise bei 180 bis 250 °C, und bei Drücken von etwa 1 bis 1050 mbar, bevorzugt bei 20 bis 350 mbar, durchgeführt. Nach dieser Methode erhält man ein Destillat (ca. 70 bis 90 % des eingesetzten Rückstandes), das in seiner Zusammensetzung in etwa der des eingesetzten Rückstandes entspricht, aber weitgehend von den Schwermetallsalzen befreit ist (Gehalt an Schwermetallsalzen im Destillat beispielsweise unter 5 ppm).

Weiterhin ist es auch möglich, beispielsweise die Schwermetallsalze von dem Rückstand durch Extraktion mit einer wässrigen Carbonsäure abzutrennen (vgl. Ullmanns Enzyklopädie der technischen Chemie, 4. Auflage, Band 8, S. 367 - 369).

Das so von den Schwermetallsalzen weitgehend befreite, aus dem Benzoesäurerückstand gewonnene Destillat wird dann ohne weitere Bearbeitung nach den für eine Seitenkettenchlorierung üblichen Bedingungen (vgl. z.B. Ullmanns Enzyklopädie der technischen Chemie, 4. Auflage, Band 9, S. 528) bei Temperaturen von etwa 80 bis 220 °C, vorzugsweise 130 bis 190 °C, vollständig chloriert. Üblicherweise wird die Chlorierung in Gegenwart von Licht (UV-Bestrahlung) durchgeführt. Es ist jedoch auch möglich, die Chlorierung im Dunklen durchzuführen.

Im allgemeinen führt man die Chlorierung mit etwa 2 bis 3,5 Mol Chlor, bevorzugt 2,01 bis 2,5 Mol) Chlor, bezogen auf 1 Mol vorhandenes Benzylbenzoat durch. Es ist aber auch unter Inkaufnahme geringerer Ausbeuten möglich, die Chlorierung mit weniger als 2 Mol Chlor, bezogen auf 1 Mol Benzylbenzoat, durchzuführen.

Aus dem bei der Chlorierung erhaltenen Produktgemisch kann dann beispielsweise durch Destillation bei Normal-, Unter- oder Überdruck reines Benzoylchlorid gewonnen werden. Im allgemeinen führt man die Destillation bei Temperaturen von etwa 30 bis 200 °C, vorzugsweise von 80 bis 130 °C, und Drücken von etwa 1 bis 1050 mbar, bevorzugt von 20 bis 150 mbar, durch. Man erhält nach dieser Methode das Benzoylchlorid in einer Ausbeute von ca. 93 %, bezogen auf das im Destillat vorhandene Benzylbenzoat.

Benzoylchlorid kann auf diese Weise in hohen Ausbeuten aus dem Benzylbenzoatanteil des Destillates gewonnen werden, wobei überraschend ist, dass die Chlorierung weder durch restliche Schwermetallsalze noch durch andere im Destillat vorhandene Verunreinigungen beeinflusst wird.

Zur Steigerung der Ausbeute an Benzoylchlorid kann man den bei der Destillation des Chlorierungsgemisches verbleibenden Rückstand, der noch ca. 5 % Benzoylchlorid neben u.a. Benzoesäure und Benzoesäureanhydrid enthält, mit Benzotrichlorid (etwa 1 bis 2 Mol, bevorzugt 1 bis 1,5 Mol, bezogen auf jeweils 1 Mol im Rückstand noch vorhandener Benzoesäure und Phenylbenzoesäure sowie entstandenen Benzoesäureanhydrids) bei Temperaturen von etwa 100 bis 220 °C, bevorzugt 130 bis 180 °C, in Gegenwart von etwa $10^{-5}$ bis $10^{-1}$ Mol, bevorzugt $10^{-4}$ bis $10^{-2}$ Mol, an Friedel-Crafts-Katalysatoren, wie Eisen(III)-chlorid, Eisen(II)-chlorid, Zink(II)-chlorid und/oder Säuren, wie Schwefelsäure und/oder Phosphorsäure, umsetzen und nach Beendigung der HCl-Gasentwicklung erneut zur Isolierung von Benzoylchlorid fraktioniert destillieren. Auf diese Weise kann man eine Ausbeute von ca. 92 %, bezogen auf im Destillat vorhandene Benzoesäure, vorhandenes Benzylbenzoat, vorhandene Phenylbenzoesäure und zugesetztes Benzotrichlorid, erzielen.

Bei dieser Ausführungsform überrascht besonders, dass sowohl die vorhandene Benzoesäure als auch das im Chlorierungsprodukt durch Umsetzung von Benzoesäure mit bereits gebildetem Benzoylchlorid entstandene Benzoesäureanhydrid trotz vorhandener Spuren von Schwermetallsalzen ebenfalls nicht kernchloriert werden und somit im Rückstand mit Benzotrichlorid zu Benzoylchlorid umgesetzt werden können, was die Ausbeute an Benzoylchlorid, bezogen auf das gesamte Destillat, beträchtlich erhöht.

Nach einer bevorzugten Ausführungsform des erfindungsgemässen Verfahrens wird das bei der Chlorierung erhaltene Produktgemisch, ohne es vorher auf Benzoyltrichlorid aufzuarbeiten, direkt weiter mit Benzotrichlorid in Gegenwart der genannten Friedel-Krafts-Katalysatoren bei Temperaturen von etwa 100 bis 220°C, bevorzugt 130 bis 180°C, umgesetzt. Man setzt dabei etwa 1 bis 2 Mol, bevorzugt 1,01 bis 1,5 Mol, Benzotrichlorid, bezogen auf jeweils 1 Mol in diesem Produktgemisch noch vorhandener Benzoesäure und Phenylbenzoesäure sowie entstandenen Benzoesäureanhydrids, ein.

Die Menge an Friedel-Crafts-Katalysatoren beträgt im allgemeinen etwa $10^{-5}$ bis $10^{-1}$ Mol, bevorzugt $10^{-4}$ bis $10^{-2}$ Mol, bezogen auf 1 Mol gebildetes Benzoylchlorid.

Nachdem die Umsetzung beendet ist, was sich am Nachlassen der HCl-Gasentwicklung bemerkbar macht, wird das Reaktionsgemisch, wie zuvor beschrieben, durch fraktionierte Destillation zur Gewinnung von Benzoylchlorid aufgearbeitet. Man erhält dabei reines Benzoylchlorid in einer Ausbeute von ca. 92%, bezogen auf eingesetzte Benzoesäure, Phenylbenzoesäure, eingesetztes Benzylbenzoat und Benzotrichlorid.

Nach dem erfindungsgemässen Verfahren gelingt es, in einfacher, wirtschaftlicher Weise in guten Ausbeuten aus dem teerartigen Rückstand, wie er bei der Benzoesäure-Herstellung anfällt, Benzoylchlorid herzustellen.

Benzoylchlorid wird in grossen Mengen zur Fabrikation von Benzophenon, Benzamid und Benzoesäureanhydrid gebraucht und dient ausserdem als Zwischenprodukt für Farbstoffe und Arzneimittel (vgl. Ullmanns Enzyklopädie der technischen Chemie, 4. Auflage, Band 8, Seite 373).

An nachfolgenden Beispielen soll das erfindungsgemässe Verfahren erläutert werden.

*Beispiel*

Bei der Herstellung von Benzoesäure anfallende Benzoesäurerückstände wurden in einem Mehrphasenwendelrohrverdampfer bei Temperaturen zwischen 200 und 250°C und Drücken von 30 bis 70 mbar eingedampft. Der Destillatanteil lag zwischen 70 bis 90%, bezogen auf den eingesetzten Rückstand. Der Kobalt- und Mangangehalt im Destillat lag unter 5 ppm.

Durchschnittliche Zusammensetzung der Benzoesäurerückstände

| | | | |
|---|---|---|---|
| Benzoesäure | 10 | bis 15 | Gew.-% |
| Benzylbenzoat | 70 | bis 7 | Gew.-% |
| Diphenyl | 0,1 | bis 0,5 | Gew.-% |
| o/m/p-Phenylbenzoesäure | 8 | bis 10 | Gew.-% |
| unbekannte Komponenten | 2 | bis 3 | Gew.-% |
| $CO^{2+}$ | | 0,3 bis 4 | Gew.ppm |
| $Mn^{2+}$ | | 0,05 bis 1 | Gew.ppm |

a) 1500 g eines wie oben beschrieben hergestellten Destillates (72,2% Benzylbenzoat, 11,6% Benzoesäure, 10,0% Phenylbenzoesäuren) werden bei 170°C im Dunkeln chloriert. Nach 3,5 Stunden hat das Gemisch durch Chloraufnahme an Gewicht ca. 380 g zugenommen.

Bei einer fraktionierten Vakuumdestillation werden 1336 g reines Benzoylchlorid erhalten (Ausbeute 93%, bezoen auf eingesetztes Benzylbenzoat). Als Rückstand verbleiben 536 g. Darin sind 5% Benzoylchlorid enthalten. Dieser Rückstand wird bei 150°C in Gegenwart von 0,1% FeCl₃ mit 450 g Benzotrichlorid versetzt (dabei wird aus Benzoesäure und Benzoesäureanhydrid Ben-zoylchlorid erhalten), um die Benzoesäureanteile in Benzoylchlorid zu überführen.

Nach Beendigung der Salzsäureentwicklung wird im Vakuum erneut fraktioniert destilliert. Es werden 449 g reines Benzoylchlorid erhalten. Dadurch ergibt sich als Ausbeute 92% (bezogen auf eingesetzte Benzoesäure, Benzylbenzoat, Phenylbenzoesäure und Benzotrichlorid). Der Rückstand beträgt 450 g.

b) 1000 g eines wie oben beschrieben hergestellten Destillates (74,2% Benzylbenzoat, 13,4% Benzoesäure, 10,0% Phenylbenzoesäure) werden bei 160°C unter Bestrahlung mit UV-Licht (Quecksilber-Dampflampe) chloriert. Nach 5 Stunden hat das Gemisch durch Chloraufnahme an Gewicht ca. 260 g zugenommen. Um noch vorhandene Benzoesäure und entstandenes Benzoesäureanhydrid in Benzoylchlorid zu überführen, werden bei 150°C in das Gemisch 330 g Benzotrichlorid eingetropft. Nach Beendigung der Salzsäureentwicklung wird die Mischung im Vakuum fraktioniert destilliert. Dabei werden 1265 g reines Benzoylchlorid (Ausbeute über 92%, bezogen auf eingesetzte Benzoesäure, Benzylbenzoat, Phenylbenzoesäure und Benzotrichlorid) erhalten. Im Destillationsrückstand verbleiben 250 g.

c) 6,2 t eines wie oben beschrieben hergestellten Destillates aus dem Rückstand einer Benzoesäurefabrikation (71,9% Benzylbenzoat, 13,0% Benzoesäure, 9% Phenylbenzoesäure, 6,1% sonstige Verbindungen) werden in einem Emaillekessel bei 160 bis 170°C im Dunkeln chloriert. Nach 37 Stunden ist die Chlorierung beendet.

1. 2000 g des Chlorierungsgemisches werden bei 150°C mit Benzotrichlorid (520 g) umgesetzt, um Benzoesäure in Benzoylchlorid überzuführen. Nach Beendigung der HCl-Gasentwicklung wird die Mischung im Vakuum fraktioniert destilliert. Dabei werden 1890 g reines Benzoylchlorid erhalten (ca. 90% Ausbeute).

2. Zu dem durch Chlorierung von 6,2 t destilliertem Rückstand erhaltenen Gemisch wird bei 150 bis 160°C Benzotrichlorid (2,0 t) langsam zugegeben, um die Benzoesäure und evtl. vorhandenes Benzoesäureanhydrid in Benzoylchlorid zu überführen. Nach Beendigung der HCl-Gasgewinnung wird eine Vakuumdestillation durchgeführt. Dabei wird reines Benzoylchlorid erhalten (7,3 t). Die Ausbeute beträgt etwa 89%.

**Patentansprüche**

1. Verfahren zur Herstellung von Benzoylchlorid, dadurch gekennzeichnet, dass man den bei der katalytischen Oxidation von Toluol mit Sauerstoff oder Sauerstoff enthaltenden Gasen in flüssiger Phase erhaltenen, hochsiedenden Rückstand von vorhandenen Schwermetallsalzen weitgehend befreit, dann den Rückstand bei erhöhten Temperaturen, gegebenenfalls unter Einwirkung von Licht, chloriert, das bei der Chlorierung erhaltene Produkt mit Benzotrichlorid in Gegenwart von Friedel-Crafts-Katalysatoren umsetzt und aus dem daraus erhaltenen Produktgemisch das Benzoylchlorid abtrennt.

2. Verfahren nach Anspruch 1, dadurch gekennzeichnet, dass man die Schwermetallsalze durch Destillation weitgehend abtrennt.

3. Verfahren nach Ansprüchen 1 und 2, dadurch gekennzeichnet, dass man die Destillation bei Temperaturen von 120 bis 330°C und Drücken von 1 bis 1050 mbar durchführt.

4. Verfahren nach Ansprüchen 1 bis 3, dadurch gekennzeichnet, dass man die Destillation bei Temperaturen von 180 bis 250°C und bei Drücken von 20 bis 350 mbar durchführt.

5. Verfahren nach Ansprüchen 1 bis 4, dadurch gekennzeichnet, dass man die Chlorierung bei Temperaturen von 80 bis 220°C durchführt.

6. Verfahren nach Ansprüchen 1 bis 4, dadurch gekennzeichnet, dass man die Chlorierung bei Temperaturen von 130 bis 190°C durchführt.

7. Verfahren nach·Ansprüchen 1 bis 6, dadurch gekennzeichnet, dass man die Chlorierung mit 2 bis 3,5 Mol Chlor, bezogen auf 1 Mol vorhandenes Benzylbenzoat, durchführt.

8. Verfahren nach Ansprüchen 1 bis 6, dadurch gekennzeichnet, dass man die Chlorierung mit 2,01 bis 2,5 Mol Chlor, bezogen auf 1 Mol vorhandenes Benzylbenzoat, durchführt.

9. Verfahren nach Ansprüchen 1 bis 8, dadurch gekennzeichnet, dass man das Benzoylchlorid durch Destillation abtrennt.

## Claims

1. Process for the preparation of benzoyl chloride, characterised in that the high-boiling residue obtained in the catalytic oxidation of toluene with oxygen or oxygen-containing gases in the liquid phase is largely freed from heavy metal salts present, then the residue is chlorinated at elevated temperatures, optionally under the action of light, the product obtained in the chlorination is reacted with benzotrichloride in the presence of Friedel-Crafts catalysts and the benzoyl chloride is separated from the product mixture thus obtained.

2. Process according to claim 1, characterised in that the heavy metal salts are largly separated off by distillation.

3. Process according to claims 1 and 2, characterised in that the distillation is carried out at temperatures from 120 to 330°C and under pressures from 1 to 1,050 mbar.

4. Process according to claims 1 to 3, characterised in that the distillation is carried out at temperatures from 180 to 250°C and under pressures from 20 to 350 mbar.

5. Process according to claims 1 to 4, characterised in that the chlorination is carried out at temperatures from 80 to 220°C.

6. Process according to claims 1 to 4, characterised in that the chlorination is carried out at temperatures from 130 to 190°C.

7. Process according to claims 1 to 6, characterised in that the chlorination is carried out using 2 to 3.5 mols of chlorine per mol of benzyl benzoate present.

8. Process according to claims 1 to 6, characterised in that the chlorination is carried out using 2.01 to 2.5 mols of chlorine per mol of benzyl benzoate present.

9. Process according to claims 1 to 8, characterised in that the benzoyl chloride is separated off by distillation.

## Revendications

1. Procédé de production de chlorure de benzoyle, caractérisé en ce qu'on débarasse largement des sels de métaux lourds présents le résidu de haut point d'ébullition obtenu en phase liquide dans l'oxydation catalytique du toluène avec l'oxygène ou des gaz contenant de l'oxygène, puis on chlore le résidu à températures élevées, éventuellement sous l'action de la lumière, on fait réagir le produit obtenu par chloration avec le chlorure de benzényle en présence de catalyseurs de Friedel-Crafts et on sépare le chlorure de benzoyle du mélange ainsi obtenu comme produit.

2. Procédé suivant la revendication 1, caractérisé en ce qu'on sépare largement les sels de métaux lourds par distillation.

3. Procédé suivant les revendications 1 et 2, caractérisé en ce qu'on conduit la distillation à des températures de 120 à 330°C et à des pressions de 1 à 1050 mbars.

4. Procédé suivant les revendications 1 à 3, caractérisé en ce qu'on conduit la distillation à des températures de 180 à 250°C et à des pressions de 20 à 350 mbars.

5. Procédé suivant les revendications 1 à 4, caractérisé en ce qu'on conduit la chloration à des températures de 80 à 220°C.

6. Procédé suivant les revendications 1 à 4, caractérisé en ce qu'on conduit la chloration à des températures de 130 à 190°C.

7. Procédé suivant les revendications 1 à 6, caractérisé en ce qu'on conduit la chloration avec 2 à 3,5 moles de chlore par rapport à 1 mole de benzoate de benzyle présent.

8. Procédé suivant les revendications 1 à 6, caractérisé en ce qu'on conduit la chloration avec 2,01 à 2,5 moles de chlore par rapport à 1 mole de benzoate de benzyle présent.

9. Procédé suivant les revendications 1 à 8, caractérisé en ce qu'on sépare le chlorure de benzoyle par distillation.